(19) Europäisches Patentamt
European Patent Office
Office européen des brevets

(11) Veröffentlichungsnummer : **0 060 381**
**B1**

(12) # EUROPÄISCHE PATENTSCHRIFT

(45) Veröffentlichungstag der Patentschrift : **15.05.85**

(51) Int. Cl.[4] : **C 07 D303/48**, C 07 D301/06

(21) Anmeldenummer : **82100395.1**

(22) Anmeldetag : **21.01.82**

(54) **Verfahren zur Herstellung von 3,3-Dimethyl-2-alkoxioxiran durch Oxidation von 1-Alkoxi-2-methylpropen.**

(30) Priorität : **12.03.81 DE 3109439**

(43) Veröffentlichungstag der Anmeldung : **22.09.82 Patentblatt 82/38**

(45) Bekanntmachung des Hinweises auf die Patenterteilung : **15.05.85 Patentblatt 85/20**

(84) Benannte Vertragsstaaten : **AT BE CH DE FR GB IT LI NL**

(56) Entgegenhaltungen :
**DE-A- 2 033 656**
**CHEMICAL ABSTRACTS, Band 79, Nr. 15, 15. Oktober 1973, Zusammenfassung 92464m, Seite 465, COLUMBUS OHIO (US)**
**JOURNAL OF ORGANIC CHEMISTRY, Band 41, Nr. 3, 6. Februar 1976, WASHINGTON (US), J.J. HAVEL u.a.: "Atomic oxygen.V. reactions of enol ethers with oxygen (3P) atoms", Seiten 513-516**

(73) Patentinhaber : **CHEMISCHE WERKE HÜLS AG - RSP Patente / PB 15 - Postfach 13 20 D-4370 Marl 1 (DE)**

(72) Erfinder : **Brockhaus, Rudolf, Dr.**
**Holsteiner Strasse 19**
**D-4370 Marl (DE)**
Erfinder : **Franke, Hans-Jürgen**
**Freiheitstrasse 13**
**D-4270 Dorsten (DE)**

**EP 0 060 381 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

3,3-Dimethyl-2-alkoxioxiran, das man auch als 1-Alkoxi-2-methylpropenoxid bezeichnet, kann man präparativ entweder durch Umsetzung der entsprechenden Halogenverbindung, beispielsweise des α-Chlorisobutyraldehyds, mit Natriummethylat oder durch Oxidation von 1-Alkoxi-2-methylpropen mit atomarem Sauerstoff herstellen (vgl. z. B. J. Org. Chem., *41*, S. 513-516 (1976)).

Beide Verfahren sind sehr aufwendig und nur zur Herstellung von Labormengen geeignet. Insbesondere die Herstellung von atomarem Sauerstoff ist für den technischen Einsatz zu aufwendig.

Die 3,3-Dimethyl-2-alkoxioxirane sind reaktionsfähige Verbindungen, die sich gezielt zu wertvollen Produkten umwandeln lassen. Es besteht daher ein wirtschaftliches Interesse, diese Verbindungen aus leicht zugänglichen Rohstoffen auf technisch einfachem Wege herzustellen.

Diese Aufgabe wird erfindungsgemäß entsprechend den Angaben der Patentansprüche gelöst.

Überraschenderweise wurde gefunden, daß man 1-Alkoxi-2-methylpropen, z. B. 1-Methoxi- oder 1-Ethoxi- oder 1-Propoxi- oder 1-Isobutoxi-2-methyl-propen in flüssiger Phase mit reinem, molekularem Sauerstoff oder mit Inertgasen verdünntem, molekularem Sauerstoff bei Temperaturen von 25 bis 70 °C, vorzugsweise von 30 bis 54 °C, zu dem entsprechenden 3,3-Dimethyl-2-alkoxioxiran oxidieren kann. Bei tieferen Temperaturen erreicht man keine befriedigende Reaktionsgeschwindigkeit.

Höhere Temperaturen begünstigen die Bildung von unverwünschten Nebenprodukten.

Der Angriff von molekularem Sauerstoff auf die olefinische Doppelbindung unter Bildung einer Epoxidverbindung ist unter den erfindungsgemäßen Bedingungen sehr überraschend. Bekannte Olefinumsetzungen in flüssiger Phase arbeiten beispielsweise mit Pd-Komplexverbindungen als Katalysator, wie bei der Oxidation von Ethylen mit Sauerstoff zu Acetaldehyd, oder mit Peroxiden in Gegenwart von Molybdänsäure, wie bei der Herstellung von Epoxiden, zum Beispiel von Propenoxid. Die erfindungsgemäße glatte Reaktion von Isobutenylethern mit molekularem Sauerstoff war daher nicht zu erwarten.

Diese oxidative Umsetzung von 1-Alkoxi-2-methylpropen mit molekularem Sauerstoff erfolgt nach folgendem Schema mit hoher Selektivität (Ausbeute bei 75 %) :

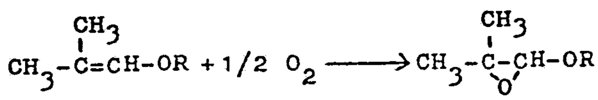

Als molekularen Sauerstoff setzt man reinen molekularen Sauerstoff oder auch mit Inerten verdünnten molekularen Sauerstoff, zum Beispiel Luft ein. Die Umsetzung erfolgt im allgemeinen bei Normaldruck, man kann jedoch auch bei Überdruck arbeiten.

Die 1-Alkoxi-2-methylpropene lassen sich auf bekannte Weise aus Isobutyraldehyd über das Acetal herstellen, indem man vom Acetal Alkohol abspaltet.

Das es für die erfindungsgemäße Oxidation nur auf die reaktive Gruppe in der Isobutenylseite des Ethers ankommt, kann auf der anderen Seite der Ethermoleküle jede Gruppe stehen, die unter den Oxidationsbedingungen stabil ist, beispielsweise eine Methyl-, Ethyl-, n-Propyl-, i-Propyl-, n-Butyl-, sec.-Butyl-, i-Butyl-, t-Butylgruppe.

Die Oxidation kann man in Abwesenheit und auch in Gegenwart von NaOH, KOH, $Ba(OH)_2$ oder Mischungen von diesen als Katalysator durchführen. Der Zusatz dieser Katalysatoren begünstigt die Selektivität der Reaktion. Diese Katalysatoren setzt man vorzugsweise in Mengen von 50 bis 200 ppm ein. Geringere Mengen an Katalysator begünstigen den Reaktionsverlauf ebenfalls, in dem genannten Bereich ist der Reaktionsverlauf optimal stabilisiert. Der Zusatz erfolgt in alkoholischer oder auch wäßriger Lösung. Die basischen Verbindungen stabilisieren das Epoxid und verhindern Folgereaktionen, beispielsweise die Bildung von Dioxanderivaten oder von α-Hydroxiacetalen.

Nach der Umsetzung trennt man das 3,3-Dimethyl-2-alkoxioxiran vom nicht umgesetzten Ausgangsprodukt durch übliche Trennmethoden, beispielsweise durch Destillation unter vermindertem Druck, ab.

Die erhaltenen 3,3-Dimethyl-2-alkoxioxirane verwendet man als Stabilisatoren für Chlorkohlenwasserstoffe. Infolge ihrer hohen Reaktionsfähigkeit setzt man sie außerdem als wertvolle Zwischenprodukte für weitere Synthesen ein. Durch Umsetzung mit Methanol erhält man beispielsweise recht beständige Acetale, die u. a. als Lösemittel geeignet sind.

Beispiel 1

In einer Oxidationsapparatur, deren Reaktionsteil aus einem Umlaufreaktor mit einem Reaktionsrohr von 40 mm Innendurchmesser und 2 000 mm Höhe und einem temperierbaren Produktumlauf besteht, begast man 2 660 g 1-Methoxi-2-methylpropen bei 34 °C mit reinem, molekularem Sauerstoff. Der Sauerstoff gelangt über eine Fritte unten in das Reaktionsrohr. Das Abgas enthält nichtumgesetzten Sauerstoff und praktisch kein CO und $CO_2$. Aus dem Reaktor ausgetragene geringe Mengen Leichtsieder, wie z. B. Aceton und Methylformiat, werden in einem Kühlsystem kondensiert.

Mit fortschreitendem Etherumsatz steigert man zur Erlangung von fast vollständigem Sauerstoffumsatz die Reaktionstemperatur auf 48 °C. Die experimentellen Daten dieses und weiterer Versuche in der beschriebenen Apparatur sind tabellarisch zusammengefaßt.

Die Analysen des Reaktionsaustrages führt man mit Hilfe der Gaschromatographie durch.

Die in der Tabelle angegebenen weiteren Beispiele führt man nach den Angaben des Beispiels 1 durch, jedoch mit den in der Tabelle angegebenen Änderungen.

Oxidation von 1-Alkoxi-2-methylpropen, experimentelle Daten

| Beispiel | Einsatz 1-Alkoxi-2-methylpropen | Oxidationsgas | Katalysator (ppm) | Reaktionstemperatur (°C) | Etherumsatz (%) | Ausbeute bezogen auf umgesetzten Ether (Mol-%) Epoxid | α-Hydroxiacetal | Dioxanderivat |
|---|---|---|---|---|---|---|---|---|
| 1 | 1-Methoxi-2-methylpropen | $O_2$ | — | 34-48 | 69 | 32,8 | 23 | 4,8 |
| 2 | " | Luft | — | 41-54 | 72 | 27 | 24,5 | 3,1 |
| 3 | " | $O_2$ | 200 NaOH | 30-46 | 67 | 75 | 5,1 | 0,7 |
| 4 | " | $O_2$ | 200 KOH | 30-45 | 76 | 69 | 5,2 | 1,5 |
| 5 | 1-Ethoxi-2-methylpropen | $O_2$ | 50 NaOH | 32-46 | 72 | 72 | 3,1 | 1,2 |

Beispiel 2 : Fahrweise mit Luft statt reinem $O_2$

Beispiel 3 : Fahrweise mit reinem $O_2$ und NaOH als Katalysator

Beispiel 4 : Fahrweise mit reinem $O_2$ und KOH als Katalysator

Beispiel 5 : Fahrweise mit reinem $O_2$ und NaOH als Katalysator, jedoch 1-Ethoxi-2-methylpropen als Einsatzstoff

**Patentansprüche**

1. Verfahren zur Herstellung von 3,3-Dimethyl-2-alkoxioxiran durch Oxidation von 1-Alkoxi-2-methylpropen, dadurch gekennzeichnet, daß man 1-Alkoxi-2-methylpropen in flüssiger Phase bei Temperaturen von 25 bis 70 °C mit molekularem Sauerstoff, gegebenenfalls in Gegenwart von Natriumhydroxid, Kaliumhydroxid und/oder Bariumhydroxid als Katalysator oxidiert.

2. Verfahren nach Anspruch 1, dadurch gekennzeichnet, daß man reinen, molekularen Sauerstoff oder mit Inertgasen verdünnten, molekularen Sauerstoff, z. B. Luft, als Oxidationsmittel einsetzt.

3. Verfahren nach den Ansprüchen 1 und 2, dadurch gekennzeichnet, daß man bei Temperaturen von 30 bis 54 °C oxidiert.

**Claims**

1. A process for the production of a 3,3-dimethyl-2-alkoxy-oxirane by oxidation of a 1-alkoxy-2-methylpropene, characterised in that a 1-alkoxy-2-methyl-propene is oxidised in liquid phase at a temperature of 25 to 70 °C with molecular oxygen, optionally in the presence of sodium hydroxide, potassium hydroxide and/or barium hydroxide as catalyst.

2. A process according to claim 1, characterised in that pure molecular oxygen or molecular oxygen diluted with inert gas, for example air, is used as oxidising agent.

3. A process according to claim 1 or 2, characterised in that the oxidation is carried out at a temperature of 30 to 54 °C.

**Revendications**

1. Procédé de préparation de 3,3-diméthyl-2-alcoxyoxiranne par oxydation de 1-alcoxy-2-méthylpropène, caractérisé par le fait que l'on oxyde le 1-alcoxy-2-méthylpropène en phase liquide, à des températures de 25 à 70 °C, au moyen d'oxygène moléculaire, éventuellement en présence d'hydroxyde de sodium, d'hydroxyde de potassium et/ou d'hydroxyde de baryum comme catalyseur.

2. Procédé selon la revendication 1, caractérisé par le fait que l'on utilise comme oxydant de l'oxygène moléculaire pur ou de l'oxygène moléculaire dilué par des gaz inertes, par exemple de l'air.

3. Procédé selon les revendications 1 et 2, caractérisé par le fait que l'on oxyde à des températures de 30 à 54 °C.